# EUROPÄISCHE PATENTSCHRIFT

(11) **EP 2 258 284 B1**
(45) Veröffentlichungstag und Bekanntmachung des Hinweises auf die Patenterteilung: **05.03.2014**
(21) Anmeldenummer: 10004910.5
(22) Anmeldetag: 10.05.2010
(51) Int. Cl.: A61B 17/16, A61B 17/3205

(54) **Wirbelsäulen-Fräser**
Spine cutter
Fraise pour colonnes vertébrales

(30) Priorität: 12.05.2009 DE 202009006792 U
(43) Veröffentlichungstag der Anmeldung: 08.12.2010
(73) Patentinhaber: Joimax GmbH, 76227 Karlsruhe (DE)
(72) Erfinder: Ries, Wolfgang, 76351 Linkenheim (DE)
(74) Vertreter: Lempert, Jost

(56) Entgegenhaltungen:
- WO-A1-93/24061
- DE-U1- 9 420 565
- DE-U1-202005 016 763
- GB-A- 2 164 277
- US-A1- 2003 170 591
- US-A1- 2009 112 261
- US-B1- 6 306 142

## Beschreibung

Die Erfindung betrifft einen Fräser für die Wirbelsäulenchirurgie, insbesondere für den Einsatz im Bereich der Halswirbelsäule, mit einem zylindrischen Fräser-Schaft und einer an dessen distalen Ende ausgebildeten Fräserzahnung sowie einen Fräser-Satz bestehend aus vorgenannten Fräsern.

Ein gattungsgemäßer Fräser ist aus der DE 20 2005 016 763 U1 bekannt und beschreibt einen Facettengelenk-Fräser, der zum Ausfräsen von Wirbelbestandteilen im Bereich der Wirbelsäule eingesetzt wird. Dieser Fräser weist einen zylindrischen Schaft und an seinem stirnseitigen, distalen Ende eine sägezahnförmig ausgebildete Zahnung auf. Die Zahnung weist vom distalen Ende nach vorne, wobei die Zähne leicht nach außen aufgeweitet sind. Die Zähne sind achsparallel gerichtet, wobei sich zwischen ihnen ebenfalls achsparallel erstreckende Nuten befinden, die radial vom Innendurchmesser der Fräserwandung bis zum Außendurchmesser reichen.

In der GB 2 164 277 A werden die Schneidkanten der Zähne durch sternförmig nach außen laufende gerade Kanten gebildet.

Es hat sich herausgestellt, dass mit den bekannten Fräsern das Knochenmaterial nicht hinreichend sanft, insbesondere aus den empfindlichen Halswirbeln, ausgefräst werden kann. Der Erfindung liegt daher die Aufgabe zugrunde, unter Vermeidung des vorgenannten Nachteils verbesserte Fräser für die endoskopische Wirbelsäulenchirurgie zu schaffen.

Die genannte Aufgabe wird bei einem Fräser der eingangs genannten Art durch die kennzeichnenden Merkmale des Anspruchs 1 gelöst.

Durch die gegenüber dem Stand der Technik veränderte Zahnung, die an der distalen Stirnseite des Fräser-Schaftes derart sternförmig ausgebildet ist, dass die Stirnseite der Zähne von der Innenwandseite des Schaftes radial nach außen weisen und insbesondere mit einem ebenen stirnseitigen Abschluss, wobei die Schneidkanten an der Kante der Stirnseite ausgebildet sind, wird ein sanfteres Fräsen bei gleichbleibender Präzision und Schneidwirkung erreicht. Der Grund der Nuten zwischen den Zähnen, der Nutgrund, ist geschlossen; zwischen den Zähnen sind also keine Schlitze ausgebildet, die sich vollständig radial durch die Schaftwandung erstrecken. Die in der Wandung des zylindrischen Schaftes verlaufenden Zahnflanken sind stirnseitig sternförmig nach außen gerichtet. Gleichwohl ist eine hohe Schärfe der Zähne der einzelnen Fräser sichergestellt.

Die Zähne sind durch Nuten getrennt, wobei zu einem distalen, dem zu fräsenden Wirbel zugewandten Ende hin sich die eingeschnittenen Nuten in axialer Richtung parabelförmig aufweiten und die Zähne zwischen Nut und Zahnwandung eine der Form der Nut entsprechende Übergangskante aufweisen. Die Nutgründe der Nuten weisen einen Winkel von mindestens 13° bis 15° zu einer Längsachse des Fräsers auf. Weiterhin sieht die Erfindung vor, dass die durch Zahnwandungen begrenzten Zähne achsparallel verlaufen und/oder dass Kanten der Zahnwandungen achsparallel zum distalen Ende hin spitz zulaufen, wobei auch vorgesehen sein kann, dass die Zahnwandungen ab der Hälfte ihrer Länge der Nuten zum distalen Ende hin spitz zulaufen. Äußerst bevorzugt liegen äußere Kanten der Zahnwandungen auf einem Außendurchmesser des Fräser-Schaftes, wobei die Schneidkanten auch stirnseitig ausgebildet sein können. Hierdurch ist ein besonders schonendes Fräsen möglich, ohne umgehendes empfindliches Gewebe zu gefährden. Ein solcher Fräser ist als Halswirbelsäulenfräser insbesondere bei der Halswirbelsäule einsetzbar. Bevorzugte Weiterbildungen eines solchen Fräsers sehen vor, dass äußere Kanten der Zähne auf einem Außendurchmesser des Fräser-Schaftes liegen und/oder dass Kanten der Zahnwandungen achsparallel zum distalen Ende hin spitz zulaufen.

In einer weiteren bevorzugten Ausführungsform weist die Zahnung abgeknickte äußere Zahnwandungen auf, wobei die Zahnwandungen bis zur Hälfte der Länge der Nuten achsparallel und zum distalen Ende hin unter einem Winkel von mindestens 30° zur Längsachse des Fräsers verlaufen. Hierbei kann vorgesehen sein, dass die Zahnung an den schräg verlaufenden Übergangskanten scharfe Schneidkanten aufweist. Somit sind in allen Ausgestaltungen des Fräsers scharfe Schneidkanten der Zähne lediglich am stirnseitigen Ende bzw. im schräg verlaufenden Bereich ausgebildet.

In einer bevorzugten Ausführungsform weist der Schaft zu einem proximalen Ende hin mindestens einen oder mehrere farbige Keramikringe zur besseren Unterscheidung von Fräsern unterschiedlicher Größe auf, wobei die hitzbeständigen Keramikringe besser haltbar und widerstandsfähiger als Farbringe aus Kunststoff sind.

In einer weiteren Ausführungsform sind am Schaft des erfindungsgemäßen Fräsers eine ringförmige Ausnehmung, womit der Fräser in einer entsprechenden Handhabungs- oder Drehvorrichtung einspannbar ist, und an einem proximalen Ende mindestens zwei rechteckige Schlitze ausgebildet, wobei durch Formschluss zur Handhabungs- oder Drehvorrichtung ein Drehmoment auf den Fräser übertragbar ist.

Vorzugsweise weist der Fräser bei einem Innendurchmesser seiner Wandung von weniger als 2 mm zwischen 4 und 8, bei einem Innendurchmesser von 2 bis 2,5 mm zwischen 5 und 10, bei einem Innendurchmesser von 3 bis 4 mm zwischen 10 und 16 und bei einem Innendurchmesser von mehr als 5mm zwischen 12 und 24 Zähne auf.

Weiter ist ein Satz von Fräsern vorgesehen, bestehend aus mindestens drei Fräsern, wobei ein erster Fräser einen Außendurchmesser besitzt, der, gegebenenfalls unter der Berücksichtigung von Toleranzen, höchstens einem Innendurchmesser eines nächst größeren Fräsers entspricht. Es erfolgt damit eine Abstimmung von Außen- und Innendurchmesser der Fräser dahingehend, dass die Fräser ineinander schiebbar sind. Dabei weist wenigstens ein Fräser eines Fräser-Satzes einen Außendurchmesser von mehr als 5mm auf.

Weitere Vorteile und Merkmale ergeben sich aus den Ansprüchen und aus der nachfolgenden Beschreibung, in der ein Ausführungsbeispiel der Erfindung unter Bezugnahme auf die Zeichnungen im Einzelnen erläutert ist. Dabei zeigt:
- Fig. 1: eine schematische vergrößerte Darstellung eines ersten Fräsers mit einem Außen- durchmesser von 2 mm;
- Fig. 1a: eine schematische Ansicht der Stirnseite des Fräsers aus Fig. 1;
- Fig. 2: eine schematische vergrößerte Darstellung eines anderen Fräsers mit einem Au- ßendurchmesser von 3,6 mm;
- Fig. 2a: eine schematische Ansicht der Stirnseite des Fräsers aus Fig. 2;
- Fig. 3: eine schematische vergrößerte Darstellung eines weiteren Fräsers mit einem Au- ßendurchmesser von 4,7 mm;
- Fig. 3a: eine schematische Ansicht der Stirnseite des Fräsers aus Fig. 3;
- Fig. 4: eine schematische Schnittzeichnung eines Fräsers im Schnitt A-A aus Fig. 2a;
- Fig. 5: eine schematische vergrößerte Darstellung eines erfindungsgemäßen Fräsers mit einer weiteren Ausgestaltung einer Zahnung; und
- Fig. 5a: eine schematische Schnittzeichnung eines erfin- dungsgemäßen Fräsers im Längsschnitt C-C aus Fig. 5.

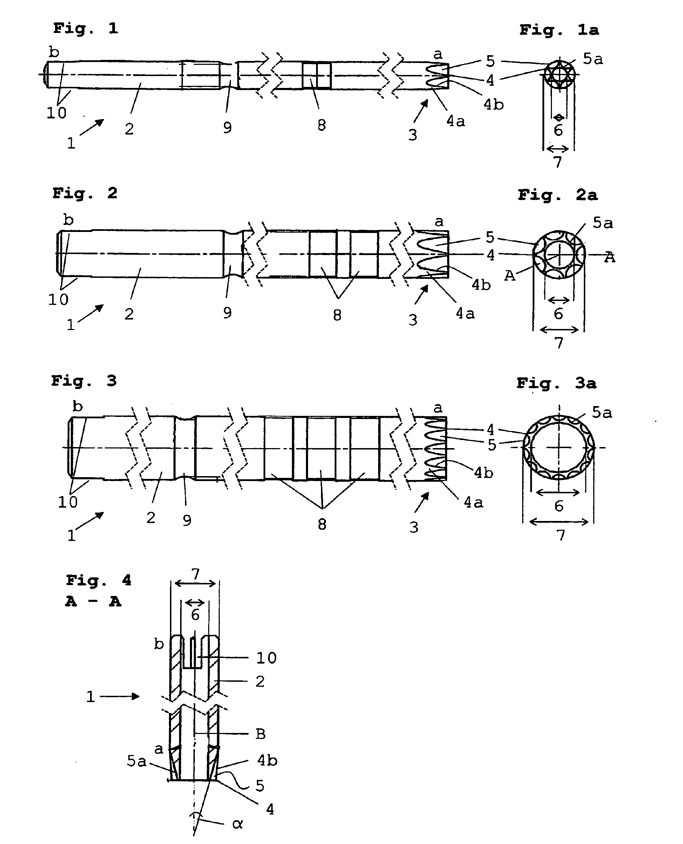

Die Fig. 1, 2 und 3 zeigen jeweils eine schematische Darstellung von Fräsern 1 und bilden zusammen einen Fräsersatz aus drei Fräsern 1 unterschiedlicher Größe. Gezackte Linien in den Figuren bedeuten eine zeichnerische Längenverkürzung zur besseren Übersicht.

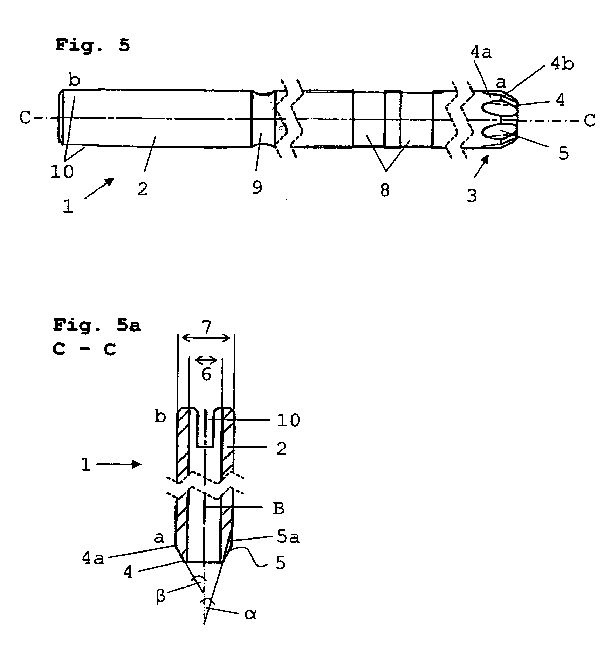

Ein Fräser 1, wie er im Ausführungsbeispiel der Fig. 1 bzw. Fig. 1a dargestellt ist, weist einen zylindrischen Schaft 2 auf, der aus einem langen Stahlröhrchen gebildet ist. Ein stirnseitiges, distales Ende a des Fräsers 1 ist als Fräserzahnung 3 ausgebildet, die aus Zähnen 4 und dazwischen liegenden Nuten 5 besteht. Die Nuten 5 sind in axialer Richtung parabelförmig in die Wandung des Schafts 2 gefräst, wobei sich die Nuten 5 zum distalen Ende a hin radial sowie axial aufweiten. Sie zeigen somit einen teilkegelförmigen Einschnitt in die Wandung des Schaftes 2. Die Zähne 4 sind aus den durch das Ausfräsen der Nuten 5 übrigbleibenden Zahnwandungen 4a gebildet, wobei die Zahnwandungen 4a zum distalen Ende a hin achsparallel spitz zulaufen. Zwischen Zahnwandung 4a und Nut 5 ist eine dem parabelförmigen Verlauf der Nut 5 entsprechende Übergangskante 4b ausgebildet. Die Übergangskante 4b kann eine Schärfung besitzen. Die Nuten 5 und die angrenzenden Zahnwandungen 4a zeigen somit eine axiale, achsparallele Hauptverlaufsrichtung, womit die gebildeten Zähne 4 auf dem äußeren Durchmesser des Schafts 2 liegen. Stirnseitig weist der Fräser 1 einen ebenen Abschluss auf. Dieser ebene Abschluss bewirkt die Ausbildung einer scharfen Schneidkante der Zähne 4 am stirnseitigen, distalen Ende a. Alternativ kann auch ein gewinkelter Abschluss vorgesehen sein, welcher im Folgenden in Fig. 5 und 5a näher erläutert wird.

Weiterhin weist der Schaft 2 des Fräsers 1 einen eingelassenen farbigen Keramikring 8 mit einer Breite von ca. 2 mm auf. Die Fräser 1 in Fig. 2 und 3 weisen hierbei nicht nur einen farbigen Keramikring 8 auf, sondern entsprechend zwei oder drei farbige Keramikringe 8. Somit ist mittels der Anzahl und der Farbe der Keramikringe 8 eine Unterscheidung der verschiedenen Fräser möglich. Des Weiteren besitzt der Schaft 2 eine ringförmige Ausnehmung 9, deren Mitte im Ausführungsbeispiel der Fig. 1 - 3 jeweils 13 mm von einem dem distalen Ende a entgegen gesetzten, proximalen Ende b entfernt ist und zur axialen Befestigung des Fräsers 1 in einer entsprechenden Handhabungs- oder Drehvorrichtung dient.

Die Fräserzahnung 3 ist weiterhin in einer schematischen stirnseitigen Ansicht in den Fig. 1a, 2a und 3a für die jeweiligen Größen des Fräsers 1 dargestellt und zeigt, dass die Zähne 4 - durch die entsprechend eingeschnittenen Nuten 5 und den ebenen Abschluss am distalen Ende a - radial nach außen, d.h. sternförmig, in einer Ebene am Fräserkopf ausgestaltet sind. Die punktsymmetrisch zur Mitte der stirnseitigen Radialebene als Kreissegment eingeschnittenen Nuten 5 weisen in der stirnseitigen Ansicht eine bestimmte Tiefe auf, welche durch eine dünne Wandung zu einem Innendurchmesser 6 begrenzt wird. Die Zähne 4 sind jeweils senkrecht zum Umfang des Fräserkopfes angeordnet, wobei deren äußere Kanten auf einem Außendurchmesser 7 des Schafts 2 liegen. Die äußeren Kanten sind durch die zulaufend ausgebildete Übergangskante 4b begrenzt, womit sich eine scharfe äußere Schneidkante der Zähne 4 am stirnseitigen distalen Ende a ergibt.

In Fig. 4 ist ein Längsschnitt durch den Fräser 1 durch die Verbindung A - A der Fig. 2a, dargestellt. Des Weiteren ist in Fig. 4, wiederum durch eine zeichnerische Verkürzung, das distale Ende a mit der Zahnung 3 mit Zähnen 4 und Nuten 5 im Längsschnitt dargestellt, wobei die Nuten 5 einen Nutgrund 5a aufweisen. Der Nutengrund 5a ändert sich hierbei über die Länge der Nuten 5 radial mit der axialen Höhe in einem bestimmten Winkel α zu einer Längsachse B hin und endet stirnseitig am distalen Ende a. Für die Fräser 1 aus Fig. 1 und 2 beträgt dieser Winkel α ca. 15° und für den Fräser 1 aus Fig. 3 beträgt der Winkel α ca. 13°. Hier ist deutlich zu erkennen, dass die Zähne 4 auf dem Außendurchmesser 7 des Schaftes 2 liegen.

Weiterhin sind am proximalen Ende b des Schaftes 2 mindestens zwei gegenüberliegende rechteckige Schlitze 10 ausgebildet, mittels derer durch Formschluss zu einer entsprechenden Handhabungs- oder Drehvorrichtung ein Drehmoment auf den Fräser 1 übertragbar ist.

Fig. 5 und 5a zeigen eine Ausgestaltung der Zahnung des erfindungsgemäßen Fräsers 1 mit einem gewinkelten Abschluss am distalen Ende a. Die Zahnung ist wiederum ausgebildet durch in axialer Richtung parabelförmig in die Wandung des Fräsers 1 eingefräste Nuten 5, wobei die dadurch geformten Zahnwandungen 4a zunächst achsparallel zum distalen Ende a hin ausgebildet sind. Ab der Hälfte der Länge der Nuten 5 sind die Zahnwandungen 4a radial nach innen abgeknickt. Die Nutgründe 5a sind zum distalen Ende a hin spitz zulaufend ausgebildet, und die Zahnwandungen 4a weisen eine nahezu konstante Breite auf. Durch den ebenen stirnseitigen Abschluss ergibt sich eine scharfe Schneidkante direkt an der Stirnseite. Eine zusätzliche Schneidkante kann im schräg verlaufenden Bereich der Zahnwandungen 4a ausgebildet sein.

Fig. 5a zeigt hierzu einen Längsschnitt nach Schnitt C-C, mit einer zeichnerischen Verkürzung zur besseren Übersicht, durch den Fräser 1 der Fig. 5. Für die hier dargestellte Variante der Zahnung 3 variiert der Nutgrund 5a der Nuten 5 ebenfalls radial mit der axialen Höhe mit dem Winkel α zur Längsachse B hin und endet stirnseitig am distalen Ende a. Die Zahnwandungen 4a verlaufen, wie schon in Fig. 5 beschrieben, nicht auf ihrer gesamten Länge achsparallel, sondern knicken ab der Hälfte der Länge der Nut 5 mit einem Winkel β zur Längsachse B des Fräsers 1 hin ab und enden ebenfalls stirnseitig am distalen Ende a. Diese Variante der Zahnung 3 weist Werte von α=13°-15° und β=30° auf.

Im vorliegenden Ausführungsbeispiel umfassen die Fräser 1 der Fig. 1 - 3 als Fräser-Satz weiterhin folgende Abmessungen des Innendurchmessers 6, des Außendurchmessers 7, der Anzahl der Zähne 4 und die Gesamtlänge des Fräsers 1:

| Innendurchmesser 6 [mm] | Außendurchmesser 7 [mm] | Anzahl Zähne 4 | Gesamtlänge Fräser 1 [mm] |
|---|---|---|---|
| 1 | 2 | 6 | 250 |
| 2,1 | 3,6 | 7 | 230 |
| 3,7 | 4,7 | 14 | 210 |

Die Fräser 1 aus Fig. 1 und 3 weisen hierbei eine Wandung mit einer Dicke von ca. 0,5 mm auf. Der Fräser in Fig. 2 weist hierzu eine Wandungsdicke von ca. 0,75 mm auf. Neben den hier aufgeführten Abmessungen sind auch Fräser 1 mit einem Außendurchmesser 7 von mehr als 5mm mit bis zu 24 Zähnen 4 mit den entsprechend hieran angepassten weiteren Abmessungen vorgesehen.

Die Fräser 1 als Fräser-Satz sind durch die aufeinander abgestimmten Durchmesser, bei denen der Außendurchmesser 7 eines dünnen Fräsers 1 mit einer Toleranz von 0,1 mm zum Innendurchmessers 6 des nächst stärkeren Fräsers 1 liegt, optimal ineinander schiebbar, so dass sie hierzu zum Aufweiten einer Aushöhlung in einem Wirbel übereinandergesetzt und/oder aufgeteilt werden können. Sie sind in ihren unterschiedlichen Größen nicht nur durch die verschiedenen Außendurchmesser 7 unterscheidbar, sondern auch durch die unterschiedliche Anzahl von farbigen Keramikringen 8 aus hitzebeständiger Keramik. Allen gemeinsam sind die ringförmige Ausnehmung 9 und der rechteckige Schlitz 10 am proximalen Ende b des Schafts 2, welche es ermöglichen, die Fräser 1 in eine entsprechende Handhabungs- oder Drehvorrichtung fest einzuspannen, durch Formschluss mit der Handhabungs- oder Drehvorrichtung ein Drehmoment zu übertragen, womit ein präzises Arbeiten in der endoskopischen Wirbelsäulenchirurgie ermöglicht wird.

### Bezugszeichenliste

- 1: Kronenfräser
- 2: Schaft
- 3: Fräserzahnung
- 4: Zahn
- 4a: Zahnwandung
- 4b: Übergangskante
- 5: Nut
- 5a: Nutgrund
- 6: Innendurchmesser
- 7: Außendurchmesser
- 8: Keramikring
- 9: ringförmige Ausnehmung
- 10: rechteckiger Schlitz
- α: Winkel
- a: distales Ende
- b: proximales Ende
- B: Längsachse

## Patentansprüche

1. Wirbelsäulen-Fräser für die Wirbelsäulenchirurgie, insbesondere für den Einsatz im Bereich der Halswirbelsäule, mit einem zylindrischen Fräser-Schaft (2) und einer an dessen distalen Ende (a) ausgebildeten Fräser-Zahnung (3), worin die FräserZahnung (3) am distalen Ende (a) des Fräser-Schaftes (2) durch Nuten in der Wandung des Fräser-Schaftes (2)
ausgebildet ist, **dadurch gekennzeichnet, dass** die Nuten sich von dem Außenradius des Fräser-Schaftes (2) zum distalen Ende (a) hin derart vertiefen und aufweiten, dass zwischen ihnen bis zum distalen Ende hin sich verschmälernde Zähne (4) mit sich vergrößernder Höhe ausgebildet sind und dass die Außen-Zahnwandungen (4a) bis zur Hälfte der Länge der Nuten (5) achsparallel verlaufen und eine Schneidkante der Fräserzahnung (3) zum distalen Ende (a) hin unter einem Winkel (β) von mindestens 30° zur Längsachse (B) des Fräsers (1) verläuft.

2. Fräser nach Anspruch 1, **dadurch gekennzeichnet, dass** zumindest der Innenumfang der Zylinderwandung des Fräserschaftes (2) am distalen Ende (a) in einer Radialebene liegt.

3. Fräser nach Anspruch 1 oder 2, **dadurch gekennzeichnet, dass** sich die zwischen den Zähnen (4) in axialer Richtung erstreckenden Nuten (5) sich zum distalen Ende (a)
hin parabelförmig aufweiten.

4. Fräser nach einem der Ansprüche 1 bis 3, **dadurch gekennzeichnet, dass** die Zähne (4) zwischen Nut (5) und Zahnwandung (4a) eine der Form der Nut (5) entsprechende Übergangskante (4b) aufweisen.

5. Fräser nach einem der Ansprüche 1 bis 4, **dadurch gekennzeichnet, dass** die Nutgründe (5a) der Nuten (5) einen Winkel (α) von mindestens 13° - 15° zu einer Längsachse (B) des Fräsers (1) aufweisen.

6. Fräser nach einem der Ansprüche 1 bis 5, **dadurch gekennzeichnet, dass** die stirnseitigen distalen Schneidkanten der Zähne (4) in einer Radialebene liegen.

7. Fräser nach Anspruch 6, **dadurch gekennzeichnet, dass** 5 die Zahnung (3) an den schräg verlaufenden Übergangskanten (4b) scharfe Schneidkanten aufweist.

8. Fräser nach einem der Ansprüche 6 oder 7, **dadurch gekennzeichnet, dass** die Zahnflanken (4a) ab der Hälfte der Länge der Nuten (5) zum distalen Ende (a) hin spitz zulaufen.

9. Fräser nach einem der Ansprüche 1 bis 8, **dadurch gekennzeichnet, dass** der Schaft (2) zu einem proximalen Ende (b) hin mindestens einen oder mehrere farbige Keramikringe (8) zur besseren Unterscheidung von Fräsern (1) unterschiedlicher Größe aufweist.

10. Fräser nach einem der Ansprüche 1 bis 9, **dadurch gekennzeichnet, dass** am Schaft (2) eine ringförmige Ausnehmung (9) zur Befestigung des Fräsers (1) in einer Handhabungs- oder Drehvorrichtung und am proximalen Ende (b) des Schafts (2) mindestens zwei gegenüberliegende rechteckige Schlitze (10) ausgebildet sind, wobei durch Formschluss zur Handhabungs- oder Drehvorrichtung ein Drehmoment auf den Fräser (1) übertragbar ist.

11. Fräser nach einem der Ansprüche 1 bis 8, **dadurch gekennzeichnet, dass** die Zähne (4) achsparallel verlaufen.

12. Fräser nach einem der vorangehenden Ansprüche, **dadurch gekennzeichnet, dass** äußere Kanten der Zähne (4) auf einem Außendurchmesser (7) des Fräser-Schaftes (2) liegen.

13. Fräser nach einem der vorangehenden Ansprüche, dass Kanten der Zahnwandungen (4a) achsparallel zum distalen Ende (a) hin spitz zulaufen.

14. Fräser-Satz, bestehend aus mindestens drei Fräsern (1) nach einem der vorangehenden Ansprüchen 1 bis 8,
**dadurch gekennzeichnet, dass** ein erster Fräser (1) einen Außendurchmesser (7) besitzt, der, gegebenenfalls unter der Berücksichtigung von Toleranzen, höchstens einem Innendurchmesser (6) eines nächst größeren Fräsers entspricht, so dass die Fräser (1) ineinander schiebbar sind.

15. Fräser-Satz nach Anspruch 14,
**dadurch gekennzeichnet, dass**
- ein dünnster Fräser (1) einen Innendurchmesser (6) von 1 mm und einen Außendurchmesser (7) von 2 mm aufweist;
- ein mittlerer Fräser (1) einen Innendurchmesser (6) von 2,1 mm und einen Außendurchmesser (7) von 3,6 mm aufweist; und
- ein stärkster Fräser (1) einen Innendurchmesser (6) von 3,7 mm und einen Außendurchmesser (7) von 4,7 mm aufweist.

## Claims

1. Spine cutter for spine surgery, especially for use in the area of the cervical spine, with a cylindrical cutter shank (2) and with cutter teeth (3) formed at the distal end (a) of said shank, wherein the cutter teeth (3) at the distal end (a) of the cutter shank (2) are formed by grooves in the wall of the cutter shank (2), **characterized in that** the grooves which become deeper and expand from the outer radius of the cutter shank (2) towards the distal end (a) such that teeth (4) narrowing towards the distal end with increasing height are formed between them und that the outer tooth walls (4a) extend parallel to the axis up to half the length of the grooves (5) and a cutting edge of the cutter teeth (3) extends at an angle (β) of at least 30° in relation to the longitudinal axis (B) of cutter (1) towards the distal end (a).

2. Cutter in accordance with claim 1, **characterized in that** at least the inner circumference of the cylinder wall of the cutter shank (2) at the distal end (a) is located in a radial plane.

3. Cutter in accordance with claim 1 or 2, **characterized in that** the grooves (5) extending between the teeth (4) in the axial direction expand parabolically towards the distal end (a).

4. Cutter in accordance with claim 1 to 3, **characterized in that** the teeth (4) have a transition edge (4b) corresponding to the shape of groove (5) between the groove (5) and the tooth wall (4a).

5. Cutter in accordance with claim 1 to 4, **characterized in that** the groove bases (5a) of the grooves (5) have an angle (α) of at least 13°-15° in relation to a longitudinal axis (B) of cutter (1).

6. Cutter in accordance with claim 1 to 5, **characterized in that** the front-side distal cutting edges of teeth (4) are located in a radial plane.

7. Cutter in accordance with claim 6, **characterized in that** the teeth (3) have sharp cutting edges at the obliquely extending transition edges (4b).

8. Cutter in accordance with claim 6 or 7, **characterized in that** the tooth flanks (4a) become pointed beginning from half the length of the grooves (5) towards the distal end (a).

9. Cutter in accordance with claim 1 to 8, **characterized in that** shank (2) has at least one or more colored ceramic rings (8) towards a proximal end (b) for the better distinction of cutters (1) of different sizes.

10. Cutter in accordance with claim 1 to 9, **characterized in that** an annular recess (9) is formed on shank (2) for fastening the cutter (1) in a handling or rotating device and at least two opposite rectangular slots (10) are formed at the proximal end (b) of shank (2), wherein a torque can be transmitted to the cutter (1) by positive-locking connection with the handling or rotating device.

11. Cutter in accordance with claim 1 to 8, **characterized in that** the teeth (4) extend parallel to the axis.

12. Cutter in accordance with one of the proceeding claims, **characterized in that** outer edges of the teeth (4) are located on an external diameter (7) of the cutter shank (2).

13. Cutter in accordance with on of the proceeding claims, **characterized in that** edges of the tooth walls (4a) become pointed parallel to the axis towards the distal end (a).

14. Set of cutters, comprising at least three cutters (1), in accordance with one of the proceeding claims 1 - 8, **characterized in that** a first cutter (1) has an external diameter (7) that corresponds, possibly by taking into account tolerances, at most to an internal diameter (6) of a next larger cutter, so that the cutters (1) can be pushed one into the other.

15. Set of cutters in accordance with claim 14, **characterized in that**
- a thinnest cutter (1) has an internal diameter (6) of 1 mm and an external diameter (7) of 2 mm;
- a medium cutter (1) has an internal diameter (6) of 2.1 mm and an external diameter (7) of 3.6 mm; and
- a thickest cutter (1) has an internal diameter (6) of 3.7 mm and an external diameter (7) of 4.7 mm.

## Revendications

1. Fraise pour colonnes vertébrales servant pour la chirurgie de la colonne vertébrale, notamment pour l'intervention dans la région de la colonne vertébrale correspondant au cou, avec un manche de fraise cylindrique (2) et un endentement de fraise (3) réalisé au niveau de son extrémité distale (a), où l'endentement de fraise (3) est réalisé au niveau de l'extrémité distale (a) du manche de fraise (2) en passant à travers les encoches prévues dans la paroi du manche de fraise (2), **caractérisée en ce que** les encoches s'approfondissent et s'élargissent en partant du rayon extérieur du manche de fraise (2) vers l'extrémité distale (a) de telle sorte que les dents (4) se rétrécissant entre elles jusqu'à l'extrémité distale sont réalisées avec une hauteur croissante et que les parois de dent extérieures (4a) s'étendent parallèlement à l'axe jusqu'à la moitié de la longueur des encoches (5) et qu'une arête tranchante de l'endentement de fraise (3) s'étend jusqu'à l'extrémité distale (a) selon un angle (β) d'au moins 30° par rapport à l'axe longitudinal (B) de la fraise (1).

2. Fraise selon la revendication 1, **caractérisée en ce qu'**au moins la circonférence intérieure de la paroi de cylindre du manche de fraise (2) repose au niveau de l'extrémité distale (a) dans un plan radial.

3. Fraise selon la revendication 1 ou 2, **caractérisée en ce que** les encoches (5) s'étendant entre les dents (4) dans la direction axiale s'élargissent en forme de parabole en direction de l'extrémité distale (a).

4. Fraise selon l'une quelconque des revendications 1 à 3, **caractérisée en ce que** les dents (4) comportent une arête de transition (4b) correspondant à la forme de l'encoche (5) entre l'encoche (5) et la paroi de dent (4a).

5. Fraise selon l'une quelconque des revendications 1 à 4, **caractérisée en ce que** les bases d'encoche (5a) des encoches (5) forment un angle (a) d'au moins 13° - 15° par rapport à un axe longitudinal (B) de la fraise (1).

6. Fraise selon l'une quelconque des revendications 1 à 5, **caractérisée en ce que** le côté avant des arêtes tranchantes distales des dents (4) repose dans un plan radial.

7. Fraise selon la revendication 6, **caractérisée en ce que** l'endentement (3) comporte des arêtes tranchantes aiguisées au niveau des arêtes de transition (4b) s'étendant en oblique.

8. Fraise selon l'une quelconque des revendications 6 ou 7, **caractérisée en ce que** les flancs de dent (4a) s'étendent en pointe à partir de la moitié de la longueur des encoches (5) jusqu'à l'extrémité distale (a).

9. Fraise selon l'une quelconque des revendications 1 à 8, **caractérisée en ce que** le manche (2) comporte, en direction d'une extrémité proximale (b), au moins une ou plusieurs bagues en céramique (8) colorées permettant une meilleure différenciation des fraises (1) de différentes tailles.

10. Fraise selon l'une quelconque des revendications 1 à 10, **caractérisée en ce qu'**un creux (9) en forme de manche est réalisé au niveau du manche (2) en vue de réaliser la fixation de la fraise (1) dans un dispositif de manipulation et pivotement et qu'au moins deux fentes (10) rectangulaires opposées sont disposées au niveau de l'extrémité proximale (b) du manche (2), un couple de rotation pouvant être transmis à la fraise (1) par le biais de la complémentarité de formes avec le dispositif de manipulation et pivotement.

11. Fraise selon l'une quelconque des revendications 1 à 8, **caractérisée en ce que** les dents (4) s'étendent parallèlement à l'axe.

12. Fraise selon l'une quelconque des revendications précédentes, **caractérisée en ce que** les arêtes extérieures des dents (4) reposent sur un diamètre extérieur (7) du manche de fraise (2).

13. Fraise selon l'une quelconque des revendications précédentes, **caractérisée en ce que** les arêtes des parois de dent (4a) s'étendent en pointe par rapport à l'extrémité distale (a), parallèlement à l'axe.

14. Jeu de fraises, composé d'au moins trois fraises (1) selon l'une quelconque des revendications 1 à 8, **caractérisé en ce qu'**une première fraise (1) possède un diamètre extérieur (7) correspondant le cas échéant, en prenant en compte les tolérances, tout au plus au diamètre intérieur (6) de la fraise venant juste après, de sorte que les fraises (1) peuvent être insérées les unes dans les autres.

15. Jeu de fraises selon la revendication 14, **caractérisé en ce que** :
- une fraise (1) plus mince présente un diamètre intérieur (6) de 1 mm et un diamètre extérieur (7) de 2 mm ;
- une fraise (1) centrale présente un diamètre intérieur (6) de 2,1 mm et un diamètre extérieur (7) de 3,6 mm ; et
- une fraise (1) plus épaisse présente un diamètre intérieur (6) de 3,7 mm et un diamètre extérieur (7) de 4,7 mm.
